# FASCICULE DE BREVET EUROPEEN

(11) **EP 1 649 415 B1**
(45) Date de publication et mention de la délivrance du brevet: **03.09.2014**
(21) Numéro de dépôt: 04767673.9
(22) Date de dépôt: 13.07.2004
(51) Int. Cl.: A61M 15/00, G06M 1/08, G06M 1/04

(54) **INDICATEUR DE DOSES AMELIORE POUR DISPOSITIF DE DISTRIBUTION DE PRODUIT FLUIDE**
VERBESSERTER DOSISINDIKATOR FÜR EINE FLÜSSIGPRODUKT-ABGABEEINRICHTUNG
IMPROVED DOSE INDICATOR FOR FLUID PRODUCT DISPENSING DEVICE

(30) Priorité: 18.07.2003 FR 0308833
(43) Date de publication de la demande: 26.04.2006
(73) Titulaire: Aptar France SAS, 27110 Le Neubourg (FR)
(72) Inventeur: STRADELLA, Fabio, I-16032 Camogli (IT); STRADELLA, Giuseppe, I-16032 Camogli (IT)
(74) Mandataire: CAPRI
(86) Numéro de dépôt international: PCT/FR2004/001844
(87) Numéro de publication internationale: WO 2005/017824

(56) Documents cités:
- WO-A-00/09187
- WO-A-01/37909
- GB-A- 1 317 315
- GB-A- 2 372 542

## Description

La présente invention concerne un indicateur de doses, ainsi qu'un dispositif de distribution de produit fluide comportant un tel indicateur.

Dans le domaine des dispositifs de distribution de produit fluide destinés à distribuer plusieurs doses, et en particulier dans le domaine des pulvérisateurs, de nombreux systèmes destinés à indiquer le nombre de doses distribuées ou le nombre de doses restant à distribuer ont été développés.

La plupart de ces systèmes présentent de nombreux inconvénients. Ainsi, ils sont généralement conçus aux moyens de plusieurs roues dentées formant des engrenages, dont le nombre dépend de la quantité de doses à compter. Par conséquent, ces compteurs ou indicateurs peuvent devenir très complexes, encombrants, et donc coûteux à fabriquer et à assembler. D'autre part, l'indication est généralement donnée par des chiffres, qui sont souvent difficiles à lire pour l'utilisateur, en particulier lorsque les dispositifs de distribution sont destinés à distribuer un grand nombre de doses, par exemple jusqu'à 200 doses. De même, tous les systèmes de compteurs ou d'indication de doses actuels ne sont pas utilisables par les personnes ayant des problèmes de vue, et notamment par les aveugles. Un autre inconvénient majeur réside dans le fait que les compteurs existants nécessitent généralement une procédure d'assemblage du dispositif de distribution qui est modifiée de par la présence du compteur, et qui diffère donc de la procédure d'assemblage habituelle. Ceci augmente la complexité du dispositif, et implique par conséquent un coût supérieur.

D'autre part, une exigence de sécurité très important est d'éviter tout risque de sous comptage, c'est-à-dire de ne pas compter une distribution totale ou partielle de produit. Pour éviter ce risque, il est nécessaire que l'actionnement du compteur soit réalisé pendant la course de l'organe de distribution, en particulier de la soupape de valve, qui se produit avant le début de l'expulsion du produit. La longueur de cette course initiale est généralement très courte, typiquement de l'ordre de 1 à 1,5 mm, et les diverses tolérances de dimension du dispositif réduisent celle-ci à quelques dixièmes de millimètres. Une course d'actionnement aussi courte rend difficile l'actionnement du compteur et peut impliquer l'utilisation de mécanismes complexes pour garantir un comptage fonctionnel.

Par ailleurs, certaines pompes ou valves peuvent être utilisées pour distribuer des doses de volume différent, en ne modifiant que la course d'actionnement du piston ou de la soupape. Il est alors généralement nécessaire de modifier les moyens d'actionnement des compteurs ou indicateurs associés pour s'adapter aux différentes courses.

Le document GB-1 317 315 divulgue un compteur comportant des moyens de compensation des tolérances prévus entre l'actionneur du compteur et le corps du dispositif logeant ledit compteur, ces moyens étant actionnés après la première utilisation du dispositif. Ce système est complexe et peu fiable car opérant pendant le fonctionnement normal du dispositif, et donc plus ou moins efficace selon la manière dont l'utilisateur actionne le dispositif.

La présente invention a pour but de fournir un indicateur de doses destiné à un dispositif de distribution de produit fluide, qui ne reproduit pas les inconvénients susmentionnés.

En particulier, la présente invention a pour but de fournir un indicateur de doses qui est simple et peu coûteux à fabriquer et à assembler, et qui peut notamment être appliqué à tous les dispositifs de distribution de produit fluide existants sans impliquer une modification de la procédure d'assemblage.

La présente invention a aussi pour but de fournir un indicateur de doses qui est de petite dimension, indépendamment du nombre de doses contenu dans le dispositif de distribution.

La présente invention a également pour but de fournir un indicateur de doses qui forme une unité complète et séparée, et qui comprend notamment les moyens d'actionnement de l'indicateur.

La présente invention a aussi pour but de fournir un indicateur de doses qui soit facile à lire pour l'utilisateur, et qui peut aussi être utilisé par des personnes ayant des problèmes de vue, et notamment par les aveugles.

La présente invention a encore pour but de fournir un indicateur de dose capable de compenser les tolérances de fabrication, en particulier indépendamment de l'actionnement et du fonctionnement dudit indicateur.

La présente invention a encore pour but de fournir un indicateur de doses qui évite tout risque de sous comptage (non prise en compte d'une dose distribuée). Plus particulièrement, la présente invention à pour but de fournir un indicateur de doses qui compte dès le début de la course d'actionnement du dispositif de distribution auquel il est associé, même si cette course est très courte, et indépendamment des tolérances de fabrication des différents composants du dispositif, ainsi qu'indépendamment de la course d'actionnement totale du dispositif.

La présente invention a donc pour objet un indicateur de doses pour dispositif de distribution de produit fluide tel que décrit dans la revendication 1. Des variantes avantageuses sont décrites dans les revendications dépendantes.

La présente invention a aussi pour objet un dispositif de distribution de produit fluide, comportant un réservoir de produit et un organe de distribution, tel qu'une pompe ou une valve, monté sur ledit réservoir, ainsi qu'un indicateur dé doses tel que défini ci-dessus.

Avantageusement, l'indicateur de dose est actionné par une partie du dispositif de distribution qui est déplacée lors de l'actionnement du dispositif, et qui coopère avec un élément de transmission dudit indicateur.

D'autres caractéristiques et avantages de la présente invention apparaîtront plus clairement au cours de la description détaillée suivante d'un mode de réalisation particulier de celle-ci, faite en référence aux dessins joints, donnés à titre d'exemples non limitatifs, et sur lesquels,
- la figure 1 est une vue schématique de côté partiellement découpée d'un dispositif de distribution de produit fluide comportant un indicateur de doses selon un mode de réalisation avantageux de la présente invention,
- la figure 2 est une vue de face, similaire à celle de la figure 1,
- la figure 3 est une vue éclatée d'un indicateur de doses selon un mode de réalisation avantageux de l'invention,
- la figure 4 est une vue similaire à celle de la figure 3, prise selon un autre angle de vue,
- la figure 5 est une vue schématique des moyens d'adaptation de l'indicateur des figures 1 à 4, en début d'assemblage du réservoir du dispositif de distribution,
- la figure 6 est une vue similaire à celle de la figure 5, en cours d'assemblage,
- la figure 7 est une vue similaire à celle des figures 5 et 6, en fin d'assemblage, et
- la figure 8 est une vue similaire à celle des figures 5 à 7, après assemblage, en position de repos du dispositif.

L'indicateur de doses A de la présente invention s'applique à tous types de dispositifs de distribution de produit fluide. Il s'applique toutefois plus particulièrement aux dispositifs de pulvérisation, et avantageusement aux dispositifs aérosols comportant une valve doseuse montée sur un récipient contenant un produit et un gaz propulseur.

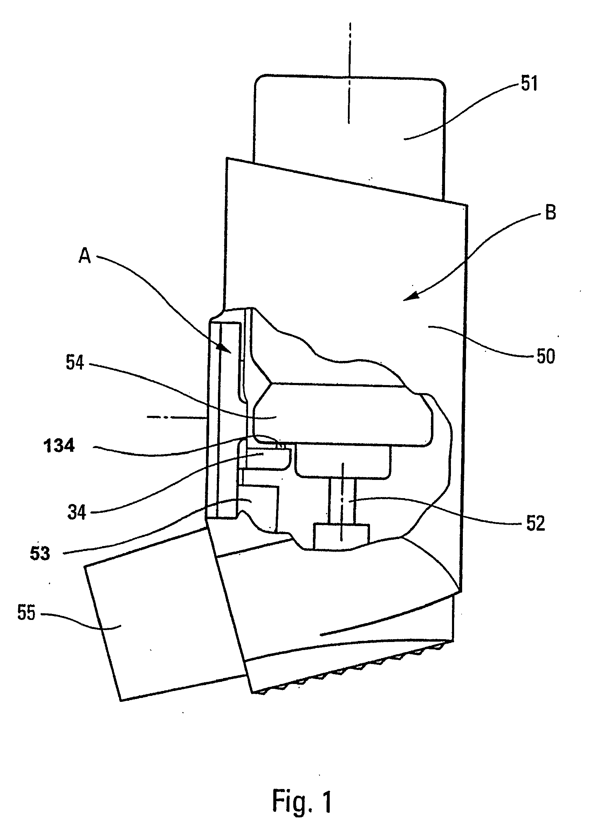

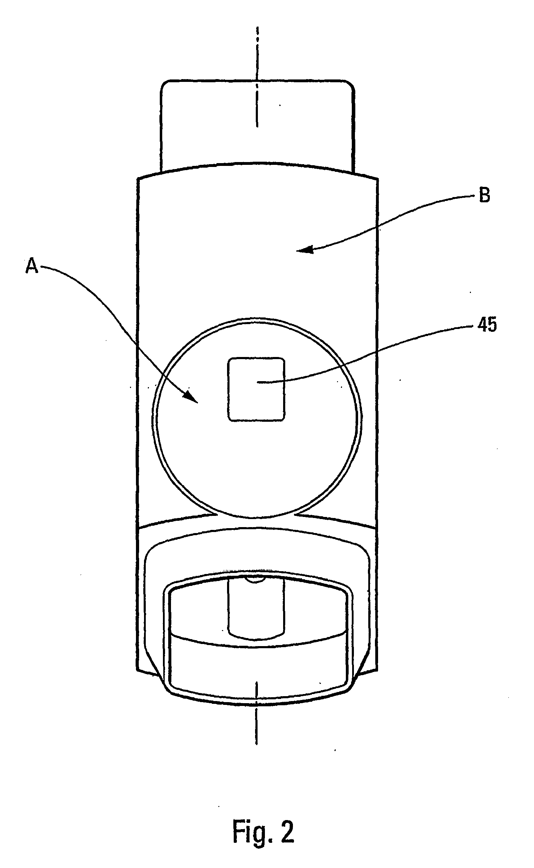

Les figures 1 et 2 représentent schématiquement un dispositif de distribution B auquel l'indicateur de doses A de la présente invention est particulièrement adapté. Ce dispositif comporte un corps 50 et un réservoir 51 sur lequel est assemblé une valve doseuse 52. L'actionnement du dispositif B étant obtenu par déplacement axial du réservoir 51 à l'intérieur du corps 50, ce déplacement entraînant une compression de la soupape de la valve 52 ce qui provoque l'expulsion d'une dose de produit à travers un orifice buccal 55. Bien entendu, la présente invention s'applique également à d'autres types de dispositifs de distribution, et notamment des dispositifs de pulvérisation du type nasal, ou des dispositifs comportant une pompe en lieu et place de la valve.

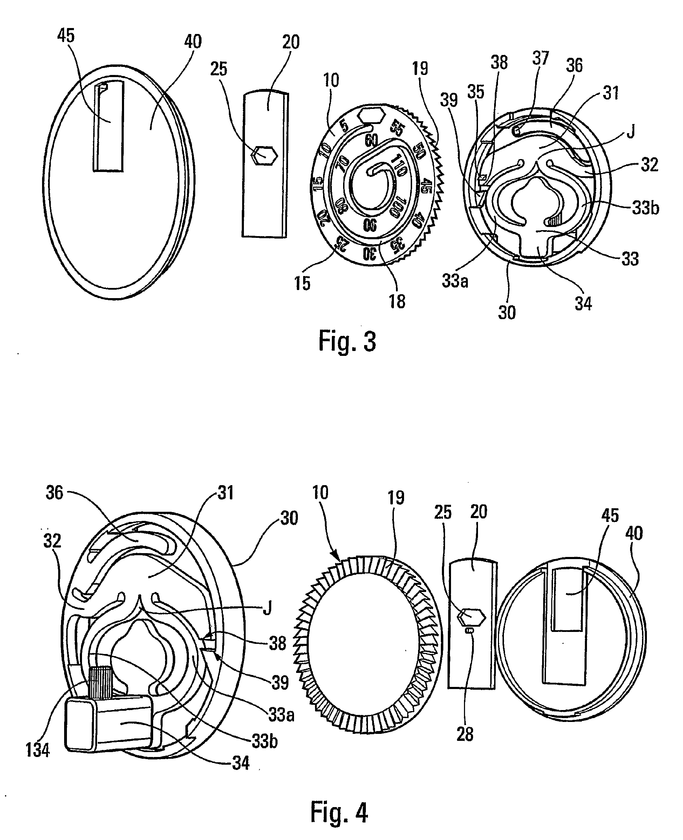

Les figures 3 et 4 représentent un indicateur de doses A qui peut notamment être utilisé avec un dispositif de distribution de produit fluide B décrit ci-dessus. Cet indicateur de doses comprend au moins un moyen de comptage rotatif qui, dans l'exemple représenté, est formé par une roue de comptage rotative 10, de préférence réalisée sous la forme d'un disque rotatif, adaptée à se déplacer en rotation autour d'un axe de rotation sensiblement perpendiculaire audit disque 10. Ce disque rotatif 10 est de préférence mince et pourvu d'un profil creux 18, qui peut avantageusement être réalisé au moyen d'une nervure ou rainure. Le disque 10 comporte en outre avantageusement une denture 19, de préférence réalisée sur sa périphérie, ladite denture 19 étant adaptée à coopérer avec des moyens d'actionnement qui sont adaptés à faire tourner ledit disque 10, et qui seront décrits plus amplement ci-après. Le disque ou roue de comptage 10 comporte également des moyens d'indication 15, qui peuvent être des nombres et/ou des symboles, et qui sont destinés à indiquer le nombre de doses distribuées ou restant à distribuer. Ces moyens d'indication 15 suivent avantageusement au moins partiellement ledit profil creux 18.

L'indicateur A représenté sur les figures peut aussi comporter avantageusement un organe translatif 20, adapté à se déplacer en translation. Cet organe translatif 20 comporte une projection 28, ou tout autre moyen équivalent, qui coopère avec ledit profil creux 18 du disque rotatif 10. Cet organe translatif 20 est de préférence réalisé sous la forme d'une plaque mince, et comporte une ouverture de visualisation 25 destinée à coopérer avec les moyens d'indication 15 du disque rotatif 10.

Selon la forme du profil creux 18, une rotation de la roue de comptage 10 peut entraîner une translation de l'organe translatif 20. Avantageusement, le profil 18 est réalisé de telle sorte que l'indication est progressive et non régulière. Par exemple, l'indicateur des figures 3 et 4 peut compter environ 120 doses, les 50 dernières étant affichées par intervalle de 5 dans l'ouverture de visualisation 25 de l'organe translatif 20, alors que les premières sont indiquées par intervalle de 10. Dans cet exemple, le profil creux 18 est d'abord en spirale, au centre du disque 10, de sorte que chaque rotation dudit disque 10 entraîne une translation dudit organe translatif 20. Lorsqu'il ne reste que 50 doses à distribuer, le profil 18 devient cylindrique, de sorte que les rotations suivantes du disque 10 ne déplacent plus l'organe translatif 20. Les chiffres d'indication 18 s'affichent alors dans l'ouverture de visualisation 25 au fur et à mesure de l'actionnement du dispositif. Après la dernière dose, un symbole spécifique 17 peut indiquer qu'il ne reste aucune dose à distribuer. D'autres progressions sont aussi envisageables.

Avantageusement, la roue de comptage 10 et l'organe translatif 20 sont disposés dans un couvercle 40, qui est de préférence également de structure mince, et qui comporte une fenêtre de visualisation 45, coopérant avec l'ouverture de visualisation 25 de l'organe translatif 20 pour permettre à l'utilisateur de visualiser les moyens d'indication 15 de la route de comptage 10

L'actionnement de l'indicateur A, et en particulier la rotation de la roue de comptage rotative 10 peut avantageusement être réalisé par des moyens d'actionnement intégrés dans ledit indicateur A. Ces moyens d'actionnement peuvent avantageusement comporter un élément d'entraînement 31 réalisé sous la forme d'une patte flexible, solidaire d'une bague 30 qui entoure ladite denture 19 du disque rotatif 10. Cette patte flexible 31 est adaptée à coopérer avec ladite denture 19 à chaque fois qu'une dose est distribuée, de préférence au moyen d'un organe d'actionnement 35, tel qu'une dent. Avantageusement, on prévoit des moyens anti-retour 36, 37 pour empêcher ledit disque rotatif 10 de tourner dans le sens inverse à celui qui lui est donné par la patte flexible 31 lors de l'actionnement. Ces moyens anti-retour peuvent comporter une patte flexible 36 supportant une dent anti-retour 37 coopérant avec la denture 19.

Les moyens d'actionnement comportent également un élément de transmission 34 qui est adapté à coopérer avec le dispositif de distribution de produit fluide B, à chaque actionnement de celui-ci, ledit élément de transmission 34 coopérant d'autre part avec ladite patte flexible 31 pour faire tourner ledit disque rotatif 10. En particulier, comme visible notamment sur la figure 1, ledit élément de transmission 34 est un épaulement solidaire de la patte flexible 31 et qui coopère avec une partie 54 du dispositif de distribution de produit fluide B qui est mobile pendant l'actionnement. Dans l'exemple représenté, il s'agit de la bague de fixation 54 de la valve doseuse 52 sur le réservoir 51. Bien entendu, et plus généralement, toute partie qui se déplace lors de l'actionnement du dispositif B est adaptée à coopérer avec l'épaulement 34 pour actionner l'indicateur de doses A.

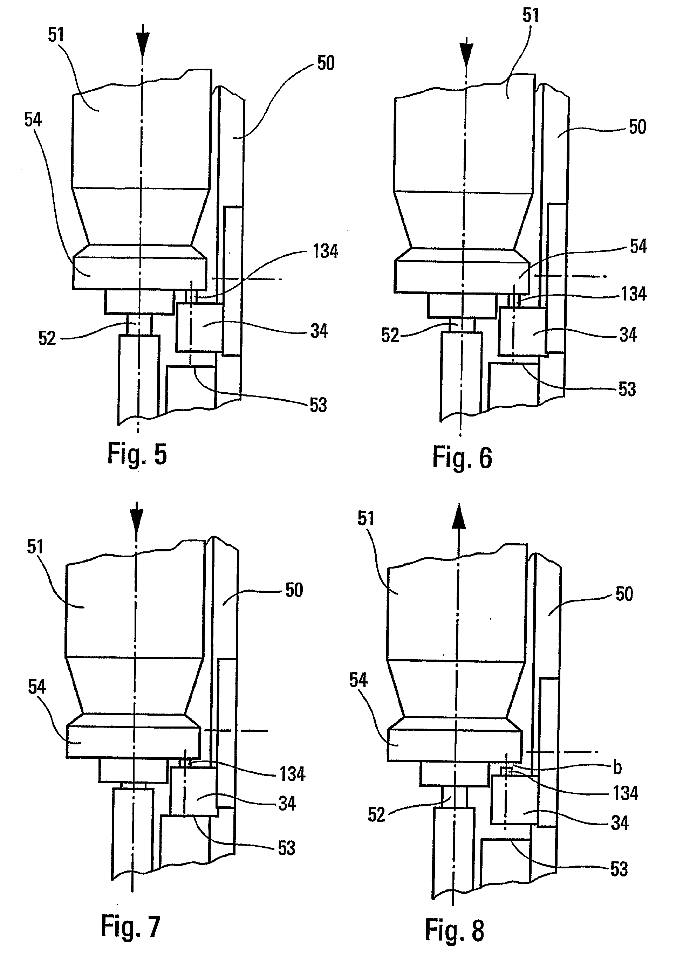

Selon l'invention, l'indicateur A comporte des moyens d'adaptation 134 permettant de compenser d'une part les tolérances de fabrication lors de l'assemblage du dispositif de distribution, et d'autre part des courses d'actionnement différentes, par exemple pour faire varier les volumes de dose. Plus précisément, lesdits moyens d'adaptation permettent de prédéterminer la course d'actionnement de l'indicateur A, en prédéterminant la distance, en position de repos, entre l'élément de transmission 34 et la partie 54 du dispositif qui coopère avec ledit élément de transmission 34 lors de l'actionnement. Cette distance est prédéterminée indépendamment des tolérances de fabrication ou de dimension des différentes parties constitutives du dispositif de distribution B et de l'indicateur A, et indépendamment de la course d'actionnement totale du dispositif de distribution. Pour ce faire, l'invention prévoit des moyens d'adaptation déplaçables et/ou déformables disposés entre l'élément de transmission 34 et la partie 54. Avantageusement, ces moyens d'adaptation sont formés par une cheville ajustable 134 solidaire de l'élément de transmission 34. Avantageusement, cette cheville 134 peut coulisser dans ledit élément de transmission 34, et la force nécessaire pour réaliser ce déplacement (et/ou une déformation correspondante) est supérieure à la force exercée lors de l'actionnement du dispositif pour distribuer du produit et actionner l'indicateur A. La cheville n'est donc déplacée que lors de l'assemblage du dispositif de distribution B, et plus particulièrement lors de l'assemblage du réservoir 51, comme visible sur les figures 5 à 8. Le corps 50 comporte une butée 53, et lors de l'assemblage, le réservoir 51 (ainsi que toute partie 54 déplaçable avec le réservoir 51) est enfoncé jusqu'à la position d'actionnement de la soupape de valve 52, avec une force suffisante pour déplacer la cheville. La figure 5 montre le début de l'assemblage. Le réservoir pousse d'abord la cheville 134 et l'élément de transmission 34 vers le bas sur les figures, en déformant les moyens d'actionnement de l'indicateur A. La figure 6 montre une position intermédiaire, avec l'élément de transmission 34 qui s'est déplacé, mais la cheville 134 qui n'a pas bougée par rapport audit élément de transmission 34. En fin de course, le fond de l'élément de transmission 34 vient contre la butée 53, et alors la cheville 134 est déplacée et/ou déformée par rapport audit élément de transmission 34 pour s'adapter à la course d'actionnement de la soupape de valve 52. La figure 8 montre le dispositif assemblé revenu en position de repos, avec la cheville 134 dans sa position ajustée. Lors des actionnements ultérieurs du dispositif B, la cheville ne sera plus déplacée, car la force exercée ne sera plus suffisante. Seule la machine d'assemblage peut provoquer le déplacement de la cheville 134. Après assemblage, l'élément de transmission 34 et la cheville 134 forment donc une unité monobloc. Comme visible sur la figure 8, une distance de sécurité « b » est définie en position de repos entre la partie 54 du dispositif B et la cheville 134 (et donc l'élément de transmission 34). Ceci peut être obtenu en fixant la distance entre la butée 53 et le fond de l'élément de transmission 34 à une valeur correspondant à la course totale d'actionnement de la soupape de valve 52 moins ladite distance de sécurité « b ». Cette distance « b » évite un actionnement intempestif ou non souhaité de l'indicateur. Les moyens d'adaptation 134 permettent donc de prédéterminer très précisément la course d'actionnement de l'indicateur A. Les tolérances de fabrication, qui influent notamment sur la course d'actionnement de la soupape de valve, sont compensées par un déplacement plus ou moins important desdits moyens d'adaptation lors de l'assemblage du dispositif. Dès le premier actionnement du dispositif, l'indicateur est donc apte à fonctionner de manière sure et fiable.

Pour éviter tout risque de sous-comptage, il est souhaitable que l'actionnement de l'indicateur se produise au début de la course d'actionnement du dispositif B, avant que l'expulsion de la dose ne commence. Pour ce faire, comme visible sur les figures 3 et 4, la patte flexible 31 peut être pourvue de deux parties flexibles 32 et 33 de flexibilité différente, la première partie 32 étant plus flexible que la seconde partie 33. La seconde partie de patte 33 supporte ledit épaulement 34, et lorsque le dispositif de pulvérisation B est actionné, la bague de fixation 54 du réservoir entraîne d'abord la partie plus flexible 32 du bras 31 à se fléchir parallèlement au disque rotatif 10, ce qui provoque la rotation dudit disque 10 au moyen de la dent d'actionnement 35 qui coopère avec la denture 19. La patte flexible 31 comporte avantageusement un élément de blocage 38 adapté à coopérer avec une butée saillante 39 solidaire de la bague 30. La distance radiale entre l'élément de blocage 38 et la butée 39 correspond avantageusement à une dent de la denture 19. Ainsi, pendant l'actionnement, l'épaulement 34 est déplacé (vers le bas sur les figures) par le dispositif de distribution B, et la partie de bras plus flexible 32 se fléchit (également vers le bas sur les figures), jusqu'à ce que l'élément de blocage 38 contacte la butée 39, comme visible sur la figure 6. Ceci entraîne la rotation sur l'équivalent d'une dent de la roue de comptage 10. La partie de bras plus flexible 32 est alors bloquée, et une poursuite de la course d'actionnement du dispositif de distribution B est possible par la flexion de la partie de bras moins flexible 33, comme visible sur la figure 7. De cette manière, on permet un actionnement de l'indicateur de doses dans la première partie de ladite course d'actionnement. Ceci élimine tout risque de non comptage d'une dose distribuée (partiellement ou totalement) en cas d'actionnement partiel du dispositif de distribution B, tout en permettant une poursuite de la course d'actionnement après comptage. La butée 39 et les moyens anti-retour 36, 37 assurent que chaque dose n'est comptée qu'une seule fois.

Avantageusement, on peut prévoir des moyens d'amplification adaptés à amplifier le déplacement de l'élément de transmission 34 au tout début de la course d'actionnement, pour que le déplacement de l'organe d'actionnement (dent) 35 soit supérieur au déplacement dudit élément de transmission 34. Pour ce faire, la seconde partie de patte 33 peut comporter une structure élastiquement déformable qui, sur les dessins, est avantageusement formée par deux branches 33a et 33b. Ces branches sont de préférence convexes et fixées d'une part à la première partie de patte 32, et d'autre part à l'élément de transmission 34. Comme visible sur les figures 3 et 4, ces branches 33a et 33b peuvent former une structure ovoïde avec deux sommets opposés, l'un formé par ladite jonction J, l'autre formé par ledit élément de transmission 34. Un déplacement de l'élément de transmission 34 provoque donc un étirement de cette structure ovoïde, qui tire sur la première partie de patte 32. L'élément de transmission 34, et donc la seconde partie de patte 33, se déplace en translation, alors que la première partie de patte 32 est déplacée en rotation. Le fait que la jonction J soit décalée par rapport à l'axe de rotation de la première partie de patte 32 provoque une amplification du déplacement de la dent 35, située de l'autre côté de la jonction J par rapport à cet axe de rotation. Dans l'exemple représenté, où la jonction est environ au centre de la première partie de patte 32, le facteur d'amplification est environ de 2. Bien entendu, en modifiant la position de la jonction J, on peut modifier le facteur d'amplificationα, sachant que α sera toujours supérieur à 1. Avantageusement, la seconde partie de patte 33 peut être guidée lors de son déplacement par des moyens appropriés (non représentés), prévus par exemple dans le corps 50 du dispositif. Avantageusement, les branches 33a et 33b reviennent élastiquement vers leur position de repos après actionnement. D'autres structures élastiquement déformables sont aussi envisageables.

Le nombre de dents sur la denture 19 et la forme du profil creux 18 de la roue de comptage 10 donnent les caractéristiques de l'indicateur de doses, et notamment le nombre de doses que cet indicateur peut compter. Le nombre maximal de doses et le mode d'affichage peuvent être variés à souhait en modifiant la structure du profil 18, les moyens d'indication, ou le nombre de dents sur la denture 19. La présente invention permet donc de réaliser des indicateurs de doses adaptés à compter un nombre quelconque de doses, sans modifier la géométrie ou la taille dudit indicateur. Comme déjà précisé précédemment, la structure dimensionnelle du présent indicateur est particulièrement faible, notamment dans la dimension de l'épaisseur, et cet indicateur A peut donc très facilement être intégré dans les dispositifs de distribution de produit fluide B existants, comme cela est visible sur les figures 1 et 2.

L'indicateur de doses de la présente invention permet de visualiser de manière simple, peu coûteuse et progressive le nombre de doses distribuées ou le nombre de doses restant à distribuer dans le dispositif. La structure de l'indicateur est très mince, indépendamment du nombre de doses qu'il doit indiquer, et il ne comporte aucune partie saillante impliquant une modification du dispositif auquel il est appliqué. Comme visible sur la figure 1, l'indicateur de doses A de la présente invention s'applique très facilement à tous les dispositifs existants, sans que ceux-ci ne doivent être modifiés. La présence de l'indicateur A ne modifie pas non plus le processus d'assemblage du dispositif B. L'indicateur peut par exemple être mis en place dans le dispositif B à travers une ouverture prévue à cet effet sur la partie frontale du corps 50 du dispositif. Un autre avantage du présent indicateur est que les moyens d'actionnement de l'indicateur sont intégrés dans celui-ci, de sorte que l'indicateur forme une unité autonome et séparée qui peut être pré-assemblée, et intégrée aisément dans n'importe quel dispositif de distribution de produit fluide. L'indicateur de doses de la présente invention garantit surtout l'actionnement dudit indicateur en tout début de course d'actionnement, notamment pendant la course initiale qui se produit avant le début de l'expulsion de la dose. Quelque soit la longueur de cette course initiale, les moyens d'adaptation de la présente invention permettent de compenser les éventuelles tolérances de fabrication, et donc de pouvoir prédéterminer la longueur de la course initiale, assurant ainsi un comptage fiable.

Bien entendu, la présente invention a été décrite en référence à un mode de réalisation particulier de celle-ci, représenté sur les dessins, mais elle n'est aucunement limitée à cette forme de réalisation particulière. Au contraire, un homme du métier peut y apporter toutes modifications sans sortir du cadre de la présente invention tel que défini dans les revendications annexées.

## Revendications

1. Indicateur de doses (A) pour dispositif de distribution de produit fluide (B), comportant au moins un moyen de comptage rotatif (10) déplaçable en rotation, ledit au moins un moyen de comptage comportant des moyens d'indication (15), indiquant le nombre de doses distribuées ou restant à distribuer, ledit au moins un moyen de comptage étant actionné par un organe d'actionnement (35) lui-même actionné par un élément de transmission (34) adapté à coopérer avec une partie (54) dudit dispositif de distribution (B) à chaque actionnement de celui-ci, ledit indicateur de doses comportant des moyens d'adaptation (134) déplaçables et/ou déformables pour prédéterminer précisément la distance au repos entre ledit élément de transmission (34) et ladite partie (54) du dispositif de distribution (B), **caractérisé en ce que** lesdits moyens d'adaptation sont disposés entre ledit élément de transmission (34) et ladite partie (54) dudit dispositif de distribution (B) et sont déplacés et/ou déformés lors de l'assemblage du dispositif de distribution (B).

2. Indicateur selon la revendication 1, dans lequel lesdits moyens d'adaptation (34) comporte un élément déformable et/ou déplaçable solidaire dudit élément de transmission (34), tel qu'une cheville (134), la force minimale nécessaire pour déplacer et/ou déformer ledit élément (134) étant supérieure à la force d'actionnement de l'indicateur, ledit élément n'étant déformé et/ou déplacé que lors de l'assemblage du dispositif de distribution (B).

3. Indicateur selon la revendication 2, dans lequel ledit au moins un moyen de comptage rotatif comporte une roue de comptage rotative (10) comportant une denture (19), ladite denture (19) coopérant avec des moyens d'actionnement (31, 34, 35) adaptés à faire tourner ladite roue rotative (10), lesdits moyens d'actionnement comportent une patte flexible (31) comportant une première partie de patte flexible (32) et une seconde partie de patte flexible (33) plus rigide que la première partie de patte (32), la première partie de patte (32) supportant une dent d'actionnement (35) adaptée à coopérer avec la denture (19) de ladite roue de comptage rotative (10) à chaque actionnement du dispositif, et la seconde partie de patte (33) supportant l'élément de transmission (34) adapté à coopérer avec ledit dispositif de distribution de produit fluide (B) à chaque actionnement de celui-ci, ladite patte flexible (31) étant solidaire d'une bague (30) entourant ladite denture (19), ladite patte flexible (31) venant coopérer avec ladite denture (19) chaque fois qu'une dose est distribuée.

4. Indicateur selon la revendication 3, dans lequel ladite bague (30) comporte des moyens anti-retour (36, 37), empêchant ledit disque rotatif (10) de tourner dans le sens opposé à celui induit par ladite patte flexible (31).

5. Indicateur selon la revendication 3 ou 4, dans lequel ladite bague (30) comporte une butée (39) adaptée à coopérer avec un élément de blocage (38) solidaire de ladite patte flexible (31) pour limiter la rotation de ladite roue de comptage rotative (10).

6. Indicateur selon la revendication 5, dans lequel la seconde partie de patte (33) plus rigide est adaptée à se fléchir à partir du moment où l'élément de blocage (38) est bloqué par les moyens de butée (39) de la bague (30).

7. Indicateur selon l'une quelconque des revendications 3 à 6, dans lequel la rotation de la roue de comptage rotative (10) est réalisée au début de la course d'actionnement du dispositif de distribution de produit fluide (B), la flexion de la seconde partie de patte (33) plus rigide permettant une poursuite de ladite course d'actionnement du dispositif de distribution de produit fluide (B) jusqu'à son terme malgré le blocage de l'élément de blocage (38) par les moyens de butée (39).

8. Indicateur selon l'une quelconque des revendications précédentes, dans lequel ledit élément de transmission (34) est un épaulement solidaire d'une patte flexible (31), et coopérant avec une partie (54) du dispositif de distribution de produit fluide (B) qui est mobile pendant l'actionnement.

9. Indicateur selon l'une quelconque des revendications précédentes, dans lequel l'indicateur (A) comporte un organe translatif (20) déplaçable en translation, les moyens d'indication (15) coopérant avec une ouverture de visualisation (25) prévue dans ledit organe translatif (20), ledit au moins un moyen de comptage rotatif comportant une roue de comptage rotative (10) comportant un profil creux (18) coopérant avec une projection (28) dudit organe translatif (20), la forme dudit profil creux (18) étant telle qu'au moins certaines rotations de ladite roue de comptage rotative (10) entraînent une translation dudit organe translatif (20), modifiant la position dudit organe translatif (20) par rapport à ladite roue de comptage (10).

10. Indicateur selon la revendication 9, dans lequel ladite roue de comptage rotative (10) et ledit organe translatif (20) sont disposés dans un couvercle (40) comportant une fenêtre de visualisation (45) coopérant avec l'ouverture de visualisation (25) de l'organe translatif (20).

11. Indicateur selon la revendication 10, dans lequel la roue de comptage rotative (10), l'organe translatif (20), les moyens d'actionnement (31, 34, 35) et le couvercle (40) forment une unité, qui peut être assemblée dans un dispositif de distribution de produit fluide (B).

12. Indicateur selon l'une quelconque des revendications 9 à 11, dans lequel lesdits moyens d'indication (15) suivent au moins partiellement ledit profil creux (18).

13. Indicateur selon l'une quelconque des revendications 9 à 12, dans lequel la forme dudit profil creux (18) est irrégulière de sorte que l'indication de dose est progressive.

14. Indicateur selon l'une quelconque des revendications 9 à 13, dans lequel ledit profil creux (18) est au moins partiellement en forme de spirale.

15. Indicateur selon l'une quelconque des revendications précédentes, dans lequel lesdits moyens d'indication (15) sont des nombres et/ou des symboles.

16. Indicateur selon l'une quelconque des revendications précédentes, dans lequel ledit indicateur comporte des moyens d'amplification, lesdits moyens d'amplification transformant un déplacement translatif (a) de l'élément de transmission (34) en un déplacement rotatif de l'organe d'actionnement (35), la projection en translation dudit déplacement rotatif étant α .a, avec α > 1.

17. Indicateur selon la revendication 2, dans lequel, après assemblage du dispositif de distribution, et en position de repos, la cheville (134) est disposé à une distance (10) « b » de la partie (54) du dispositif de distribution (B) destinée à actionner l'indicateur (A).

18. Dispositif de distribution de produit fluide (B) comportant un réservoir de produit (51) et un organe de distribution (52), tel qu'une pompe ou une valve, monté sur ledit réservoir (51), **caractérisé en ce qu'**il comporte un indicateur de doses (A) selon l'une quelconque des revendications précédentes.

19. Dispositif selon la revendication 18, dans lequel l'indicateur de doses (A) est actionné par une partie (54) du dispositif de distribution (B) qui est déplacée lors de l'actionnement du dispositif (B), et qui coopère avec l'élément de transmission (34) dudit indicateur (A).

## Patentansprüche

1. Dosisanzeiger (A) für eine Ausgabevorrichtung für ein fluides Produkt (B), aufweisend mindestens ein drehbares Zählmittel (10), das drehverschiebbar ist, wobei das mindestens eine Zählmittel Anzeigemittel (15) aufweist, die die Anzahl an ausgegebenen oder noch auszugebenden Dosen anzeigt, wobei das mindestens eine Zählmittel durch ein Betätigungselement (35) betätigt wird, welches selber durch ein Übertragungselement (34) betätigt wird, das dazu geeignet ist, mit einem Teil (54) der Ausgabevorrichtung (B) bei jeder Betätigung von dieser zusammen zu wirken, wobei der Dosisanzeiger Anpassungsmittel (134) aufweist, die verschiebbar und/oder verformbar sind, um den Abstand zwischen dem Übertragungselement (34) und dem Teil (54) der Ausgabevorrichtung (B) im Ruhezustand genau vorzubestimmen, **dadurch gekennzeichnet, dass** die Anpassungsmittel zwischen dem Übertragungselement (34) und dem Teil (54) der Ausgabevorrichtung (B) angeordnet sind und bei der Montage der Ausgabevorrichtung (B) verschoben und/oder verformt werden.

2. Anzeiger nach Anspruch 1, wobei die Anpassungsmittel (34) ein verformbares und/oder verschiebbares Element in einem Stück mit dem Übertragungselement (34), wie einen Stift (134), aufweisen, wobei die kleinste nötige Kraft zum Verschieben und/oder Verformen des Elements (134) größer ist als die Betätigungskraft des Anzeigers, wobei das Element nur bei der Montage der Ausgabevorrichtung (B) verformt und/oder verschoben wird.

3. Anzeiger nach Anspruch 2, wobei das mindestens eine drehbare Zählmittel ein drehbares Zählrad (10) aufweist, welches eine Verzahnung (19) aufweist, wobei die Verzahnung (19) mit den Betätigungsmitteln (31, 34, 35), die dazu geeignet sind, das drehbare Rad (10) in Drehung zu versetzen, zusammenwirkt, wobei die Betätigungsmittel eine flexible Lasche (31) aufweisen, welche einen ersten Teil der flexiblen Lasche (32) und einen zweiten Teil der flexiblen Lasche (33), der steifer ist als der erste Teil der Lasche (32), aufweist, wobei der erste Teil der Lasche (32) einen Betätigungszahn (35) trägt, der dazu geeignet ist, bei jeder Betätigung der Vorrichtung mit der Verzahnung (19) des drehbaren Zählrads (10) zusammen zu wirken, und der zweite Teil der Lasche (33) das Übertragungselement (34) trägt, das dazu geeignet ist, mit der Ausgabevorrichtung für ein fluides Produkt (B) bei jeder Betätigung von dieser zusammen zu wirken, wobei die flexible Lasche (31) mit einem Ring (30), der die Verzahnung (19) umgibt, einstückig gebildet ist, wobei die flexible Lasche (31) jedes Mal, wenn eine Dosis ausgegeben wird, mit der Verzahnung (19) zusammenwirkt.

4. Anzeiger nach Anspruch 3, wobei der Ring (30) Rückführsperrmittel (36, 37) aufweist, die verhindern, dass sich die Drehscheibe (10) in eine Richtung entgegengesetzt zu der von der flexiblen Lasche (31) induzierten Richtung dreht.

5. Anzeiger nach Anspruch 3 oder 4, wobei der Ring (30) einen Anschlag (39) aufweist, der dazu geeignet ist, mit einem mit der flexiblen Lasche (31) in einem Stück ausgebildeten Sperrelement (38) zusammen zu wirken, um die Drehung des drehbaren Zählrads (10) zu begrenzen.

6. Anzeiger nach Anspruch 5, wobei der zweite, steifere Teil der Lasche (33) dazu geeignet ist, ab dem Moment nachzugeben, ab dem das Sperrelement (38) durch die Anschlagmittel (39) des Rings (30) blockiert ist.

7. Anzeiger nach einem der Ansprüche 3 bis 6, wobei die Drehung des drehbaren Zählrads (10) zu Beginn des Betätigungshubs der Ausgabevorrichtung für ein fluides Produkt (B) realisiert wird, wobei das Nachgeben des zweiten, steiferen Teils der Lasche (33) trotz der Sperrung des Sperrelements (38) durch die Anschlagmittel (39) eine Weiterführung des Betätigungshubs der Ausgabevorrichtung für ein fluides Produkt (B) bis zu seinem Ende erlaubt.

8. Anzeiger nach einem der vorhergehenden Ansprüche, wobei das Übertragungselement (34) ein mit einer flexiblen Lasche (31) in einem Stück ausgebildeter Absatz ist und mit einem Teil (54) der Ausgabevorrichtung für ein fluides Produkt (B) zusammenwirkt, das während der Betätigung beweglich ist.

9. Anzeiger nach einem der vorhergehenden Ansprüche, wobei der Anzeiger (A) eine Verschiebeeinrichtung (20) aufweist, die translatorisch verschiebbar ist, wobei die Anzeigemittel (15) mit einer Sichtöffnung (25) zusammenwirken, die in der Verschiebeeinrichtung (20) vorgesehen ist, wobei das mindestens eine drehbare Zählmittel ein drehbares Zählrad (10) aufweist, welches ein Hohlprofil (18) aufweist, das mit einem Vorsprung (28) der Verschiebeeinrichtung (20) zusammenwirkt, wobei die Form des Hohlprofils (18) derart ist, dass zumindest gewisse Drehungen des drehbaren Zählrads (10) eine translatorische Verschiebung der Verschiebeeinrichtung (20) nach sich ziehen, die die Position der Verschiebeeinrichtung (20) in Bezug auf das Zählrad (10) ändert.

10. Anzeiger nach Anspruch 9, wobei das drehbare Zählrad (10) und die Verschiebeeinrichtung (20) in einem Deckel (40) angeordnet sind, der ein Sichtfenster (45) aufweist, das mit der Sichtöffnung (25) der Verschiebeeinrichtung (20) zusammenwirkt.

11. Anzeiger nach Anspruch 10, wobei das drehbare Zählrad (10), das Verschiebeelement (20), die Betätigungsmittel (31, 34, 35) und der Deckel (40) eine Einheit bilden, die in eine Ausgabevorrichtung für ein fluides Produkt (B) montiert werden kann.

12. Anzeiger nach einem der Ansprüche 9 bis 11, wobei die Anzeigemittel (15) zumindest teilweise dem Hohlprofil (18) folgen.

13. Anzeiger nach einem der Ansprüche 9 bis 12, wobei die Form des Hohlprofils (18) ungleichmäßig ist, so dass die Dosisanzeige schrittweise erfolgt.

14. Anzeiger nach einem der Ansprüche 9 bis 13, wobei das Hohlprofil (18) zumindest teilweise spiralförmig ist.

15. Anzeiger nach einem der vorhergehenden Ansprüche, wobei die Anzeigemittel (15) Zahlen und/oder Symbole sind.

16. Anzeiger nach einem der vorhergehenden Ansprüche, wobei der Anzeiger Verstärkungsmittel aufweist, wobei die Verstärkungsmittel eine translatorische Verschiebung (a) des Übertragungselements (34) in eine Drehverschiebung des Betätigungselements (35) umwandelt, wobei die Verschiebestrecke der Drehverschiebung α .a ist, wobei α > 1.

17. Anzeiger nach Anspruch 2, wobei nach der Montage der Ausgabevorrichtung in der Ruheposition der Stift (134) in einem Abstand (10) "b" des Teils (54) der Ausgabevorrichtung (B) zur Betätigung des Anzeigers (A) angeordnet ist.

18. Ausgabevorrichtung für ein fluides Produkt (B), aufweisend ein Produktbehältnis (51) und eine Ausgabeeinrichtung (52), wie eine Pumpe oder ein Ventil, die auf dem Behältnis (51) montiert ist, **dadurch gekennzeichnet, dass** sie einen Dosisanzeiger (A) nach einem der vorhergehenden Ansprüche aufweist.

19. Vorrichtung nach Anspruch 18, wobei der Dosisanzeiger (A) durch ein Teil (54) der Ausgabevorrichtung (B) betätigt wird, welches bei der Betätigung der Vorrichtung (B) verschoben wird und mit dem Übertragungselement (34) des Anzeigers (A) zusammenwirkt.

## Claims

1. A dose indicator (A) for a fluid product dispensing device (B), including at least one rotary counting means (10) capable of being rotated, said at least one counting means including indicating means (15), showing the number of doses dispensed or remaining to be dispensed, said at least one counting means being actuated by an actuating member (35) which itself is actuated by a transmission element (34) suitable to cooperate with a part (54) of said dispensing device (B) at each actuation of the latter, said dose indicator including adaptation means (134) movable and/or deformable in order to accurately predetermine the distance at rest between said transmission element (34) and said part (54) of dispensing device (B), **characterised in that** said adaptation means are located between said transmission element (34) and said part (54) of said dispensing device (B) and are movable and/or deformable during assembly of the dispensing device (B).

2. An indicator according to claim 1, in which said adaptation means (34) includes a deformable and/or movable element attached to said transmission element (34), such as a peg (134), the minimum force necessary to move and/or deform said element (134) being greater than the force required to actuate the indicator, where said element is deformed and/or moved only during assembly of the dispensing device (B).

3. An indicator according to claim 2, in which said at least one rotary counting means includes a rotary counting wheel (10) fitted with teeth (19), said teeth (19) cooperating with actuating means (31, 34, 35) that are suitable to turn said rotary wheel (10), where said actuating means include a flexible tab (31) that includes a first flexible tab part (32) and a second flexible tab part (33) which is more rigid than the first tab part (32), the first tab part (32) bearing an actuating tooth (35) designed to cooperate with the teeth (19) of said rotary counting wheel (10) at each actuation of the device, and the second tab part (33) supporting the transmission element (34) suitable to cooperate with said fluid product dispensing device (B) at each actuation of the latter, said flexible tab (31) being attached to a ring (30) surrounding said teeth (19), with said flexible tab (31) cooperating with said teeth (19) each time a dose is dispensed.

4. An indicator according to claim 3, in which said ring (30) includes non-return means (36, 37), preventing said rotary disk (10) from turning in the direction opposite to that induced by said flexible tab (31).

5. An indicator according to claim 3 or 4, in which said ring (30) includes a stop (39) suitable to cooperate with a blocking element (38) attached to said flexible tab (31) so as to limit the rotation of said rotary counting wheel (10).

6. An indicator according to claim 5, in which the second, more rigid tab part (33) is designed to flex from the moment when the blocking element (38) is blocked by the stop means (39) of the ring (30).

7. An indicator according to any of claims 3 to 6, in which the rotation of the rotary counting wheel (10) is effected at the beginning of the actuating stroke of the fluid product dispensing device (B), with the flexing of the second, more rigid tab part (33) allowing continuation of said actuating stroke of the fluid product dispensing device (B) for its full distance, despite the blocking of the blocking element (38) by the stop means (39).

8. An indicator according to any of the preceding claims, in which said transmission element (34) is a shoulder attached to a flexible tab (31), and cooperating with a part (54) of the fluid product dispensing device (B) which is movable during its actuation.

9. An indicator according to any of the preceding claims, in which the indicator (A) includes a linearly movable member (20) which can be moved linearly, with the indicating means (15) cooperating with a viewing opening (25) provided in said linearly movable member (20), where said at least one rotary counting means includes a rotary counting wheel (10) with a hollow profile (18) that cooperates with a projection (28) of said linearly movable member(20), the shape of said hollow profile (18) being such that at least some rotations of said rotary counting wheel (10) result in a linear motion of said linearly movable member (20), changing the position of said linearly movable member (20) in relation to said counting wheel (10).

10. An indicator according to claim 9, in which said rotary counting wheel (10) and said linearly movable member (20) are placed in cover (40) that includes a viewing window (45) which cooperates with the viewing opening (25) of the linearly movable member (20).

11. An indicator according to claim 10, in which the rotary counting wheel (10), the linearly movable member (20), the actuating means (31, 34, 35) and the cover (40) form a unit which can be mounted in a fluid product dispensing device (B).

12. An indicator according to any of claims 9 to 11, in which said indicating means (15) follow said hollow profile at least partially (18).

13. An indicator according to any of claims 9 to 12, in which the shape of said hollow profile (18) is irregular so that dose indication is progressive.

14. An indicator according to any of claims 9 to 13, in which said hollow profile (18) is at least partially in the shape of a spiral.

15. An indicator according to any of the preceding claims, in which said indicating means (15) are numbers and/or symbols.

16. An indicator according to any of the preceding claims, in which said indicator includes amplification means, with said amplification means converting linear movement (a) of the transmission element (34) into rotary movement of the actuating member (35), the projection in linear motion of said rotary movement being α.a, where α > 1.

17. An indicator according to claim 2, in which, after assembly of the dispensing device, and in the rest position, the peg (134) is located at a distance "b" from the part (54) of the dispensing device (B) intended to actuate the indicator (A).

18. A fluid product dispensing device (B) that includes a product reservoir (51) and a dispensing member (52), such as a pump or a valve mounted on said reservoir (51), **characterised in that** it includes a dose indicator (A) according to any of the preceding claims.

19. A device according to claim 18, in which the dose indicator (A) is actuated by a part (54) of the dispensing device (B) which is moved during the actuation of the device (B), and which cooperates with the transmission element (34) of said indicator (A).
